# EUROPEAN PATENT SPECIFICATION

(11) **EP 3 355 968 B1**
(45) Date of publication and mention of the grant of the patent: **12.05.2021**
(21) Application number: 16774503.3
(22) Date of filing: 29.09.2016
(51) Int. Cl.: A61M 5/28, A61M 5/31

(54) **MULTI CHAMBER SYRINGE UNIT**
MEHRKAMMERSPRITZENEINHEIT
UNITÉ DE SERINGUE À CHAMBRES MULTIPLES

(30) Priority: 02.10.2015 EP 15188184
(43) Date of publication of application: 08.08.2018
(73) Proprietor: F. Hoffmann-La Roche AG, 4070 Basel (CH)
(72) Inventor: LÜMKEMANN, Jörg, 79540 Lörrach (DE); MAHLER, Hanns-Christian, 79541 Lörrach (DE); WERK, Tobias, 4125 Riehen (CH)
(74) Representative: Latscha Schöllhorn Partner AG
(86) International application number: PCT/EP2016/073272
(87) International publication number: WO 2017/055462

(56) References cited:
- WO-A1-2010/130810
- WO-A1-2014/203133
- WO-A2-2008/007370
- GB-A- 1 464 797
- JP-A- 2007 260 349
- US-A- 5 364 350
- US-A1- 2005 164 981
- US-A1- 2007 036 745
- US-A1- 2007 060 876
- US-A1- 2007 212 385
- US-A1- 2008 255 521
- US-A1- 2008 275 387
- US-A1- 2008 281 271
- US-A1- 2009 092 677
- US-A1- 2012 100 103
- US-A1- 2012 156 164
- US-A1- 2015 057 608

## Description

### Technical Field

The present invention relates to a multi chamber syringe unit according to the preamble of independent claim 1 and more particularly to a method of preparing an according multi chamber syringe unit and a therapeutic method using such a multi chamber syringe unit.

Multi chamber syringe units can comprise a longitudinal body, a separating element and a bypass arrangement. Thereby, the body has a side wall, a distal end side, a proximal end side opposite to the distal end side, an interior limited by the side wall between the distal end side and the proximal end side and a distal opening arranged in the distal end side for providing a liquid out of the body. The separating element is arranged in the interior of the body such that a distal chamber and a proximal chamber are formed in the interior of the body, wherein the separating element seals the distal chamber from the proximal chamber. The bypass arrangement is provided in the side wall of the distal chamber of the body. Such multi chamber syringe units can be used for providing and applying one or plural pharmaceutical substances or mixtures to a patient.

### Background Art

Many pharmaceutical products are applied to patients in liquid form wherein injecting the product often is necessary when administration needs to be very quick, e.g. in case of an emergency, or the bioavailability via the gastro intestinal tract or other routes of administration is not sufficiently given. Particularly for subcutaneous, intramuscular, intradermal, intravitreal or other injections, the pharmaceutical substances are often provided in pre-filled syringes wherein staked-in needle prefilled syringes but also other syringes and cartridges or injectors have been shown to be comparably convenient to handle and use. In pre-filled syringes the pharmaceutical substance is provided in the interior of the syringe in a liquid form ready for being applied. Like this, the user receives a ready-to-inject syringe without the requirement to fill the pharmaceutical solution into the syringe or sometimes even without the need to manually assemble the needle to the syringe body. The occurrence of injuries or inappropriate handling during application can thereby be substantially lowered.

For pharmaceutical substances being unstable in liquid form such as many biopharmaceutical substances it is also known to provide the pharmaceutical substance in a freeze-dried or lyophilized form in which it is essentially more stable and robust compared to its liquid form. For delivering and applying such pharmaceutical substances specific double chamber syringes are used wherein one chamber houses the lyophilized pharmaceutical substance and the other a suitable diluent. Before being injected the lyophilized pharmaceutical substance is then reconstituted or solved in a diluent or liquid. Such reconstitution of the pharmaceutical substance inside the double chamber syringe is performed by transferring the diluent into the chamber of the lyophilized pharmaceutical substance and mixing the two. In liquid form, the pharmaceutical substance is then injected and delivered to the patient.

However, in many medical therapies it is required to apply a plurality of pharmaceutical substances. Thereby, it often is required to inject two or more substances which for stability reasons can only be mixed together shortly before administration. In these cases the use of pre-filled syringes often is not possible. Typically, the pharmaceutical substances are mixed before administration, provided into a syringe and then injected. Since such mixing and provision into the syringe has to be very accurate in order to assure adequate mixing ratio and a contamination free administration it is typically required that a skilled person such as a pharmacist prepares such a dosage form and a skilled person such as a doctor or a nurse performs administration.

Furthermore, in therapies requiring application of plural pharmaceutical substance it is also often required to sequentially inject two or more substances. For example, in some applications it is desired in a first step to inject an anaesthetic and in a second step to inject a therapeutic ingredient. In such applications, two injections have to be performed which, particularly when being performed by untrained persons such as the patients themselves, doubles the risk of a misapplication. For example, the risk of an infection due to contamination is considerably higher if plural injections have to be performed. In this context, WO 2008/007370 A2 describes a double chamber syringe for sequentially sequentially administering two liquids by one single injection. US 2012/0100103 A1 describes an in situ forming injectable hydrogel and WO 2014/203133 A1 describes a hyaluronidase from S. koganeiensis.

Therefore, there is need for a device or method allowing for adequately administering plural pharmaceutical substances by injection.

### Disclosure of the Invention

According to the invention this need is fulfilled by a syringe as it is defined by the features of independent claim 1. Preferred embodiments are subject of the dependent claims.

In particular, the invention is a multi chamber syringe unit comprising a longitudinal body with a side wall, a distal end side, a proximal end side opposite to the distal end side, an interior limited by the side wall between the distal end side and the proximal end side and a distal opening arranged in the distal end side for providing a liquid out of the body. The multi chamber syringe unit further has a separating element arranged in the interior of the body such that a distal chamber and a proximal chamber are formed in the interior of the body, wherein the separating element seals the distal chamber from the proximal chamber. The separating element can be a plunger or a middle plunger or any other temporary separation element. They can be made of an elastic material such as rubber or an elastic plastic material such as butyl.

The multi chamber syringe unit is equipped with a bypass arrangement being provided in the side wall of the distal chamber of the body. It can, e.g., be embodied by a bulge in the side wall which is dimensioned to allow liquid to pass the separation element when the being positioned adjacent to the bulge. Or, alternatively, it can be embodied by a similarly dimensioned groove or channel in the side wall. Also, other arrangements that allows liquid to bypass or flow through the separation element separating the plurality of chambers are possible.

The multi chamber syringe unit particularly comprises a first pharmaceutical liquid arranged in the distal chamber of the body and a second pharmaceutical liquid arranged in the proximal chamber of the body.

The multi chamber syringe unit can be a double chamber syringe or double chamber syringe unit. For many advantageous applications it can be a staked-in needle pre-filled double chamber syringe.

In an embodiment not covered by the claims, the multi chamber syringe unit is adapted for mixing the first pharmaceutical liquid with the second pharmaceutical liquid before injection. In particular, such embodiments allow injecting the two pharmaceutical liquids concomitantly.

For allowing such concomitant injection, the bypass arrangement can be located and shaped such that when the multi chamber syringe unit is activated, typically by pushing an activation rod, the second pharmaceutical liquid is transferred from the second chamber into the first chamber. For example, pushing the activation rod may cause an increase of pressure inside the second chamber such that the separating element is moved towards the distal end of the body. When it is located at the bypass arrangement, the second pharmaceutical liquid passes into the distal chamber. There it is mixed with the first pharmaceutical liquid. By further advancing the activation rod, the mixed first and second pharmaceutical liquids are pushed out of the distal opening of the body typically into a needle connected to the distal opening.

Alternatively, the multi chamber syringe unit can be arranged for sequentially providing the first and second pharmaceutical liquids. For this purpose, the multi chamber syringe unit is adapted for, upon activation, initially providing the first pharmaceutical liquid out of the distal opening of the body and afterwards providing the second pharmaceutical liquid out of the distal opening of the body.

According to the invention, for this, the bypass arrangement is located adjacent to the distal end side of the body. In this connection the term "adjacent" can be referred to as close to the distal end side as possible or feasible. The closer the bypass arrangement is located at the distal end side the less first pharmaceutical liquid is left in the interior of the body when the second pharmaceutical liquid passes via the bypass arrangement. Thereby, the body and the separating element preferably are arranged such that the distal chamber is essentially emptied when the separating element is moved to or located at the bypass arrangement.

In use of these embodiments allowing for a sequential provision of the first and second pharmaceutical liquids, an activation rod may be pushed into the interior of the body which causes an increase of the pressure inside the proximal chamber. This causes the separating element to be moved towards the distal end side. Thereby, the first pharmaceutical liquid is forced out of the distal opening of the body. When the separating element is located at the bypass arrangement, the distal chamber is essentially empty. In this connection the term "essentially empty" can still allow some residues of the first pharmaceutical liquid. In particular, the space between the separating element and the distal end side may still contain some few first pharmaceutical substance left when the separating element is at the bypass arrangement. By further advancing the activation rod the second liquid passes the separating element and is provided out of the distal opening of the body.

Thus, as the need may be, the multi chamber syringe allows for conveniently and safely providing a first and a second pharmaceutical liquid out of the body. In particular, the first and the second pharmaceutical liquid can be injected in a predefined and preferred manner, i.e. sequentially. Like this, the multi chamber syringe unit allows for adequately administering plural pharmaceutical liquids by injection.

In a first preferred embodiment, the first pharmaceutical liquid is an anaesthetic and the second pharmaceutical liquid comprises a therapeutic active ingredient. Thereby, the anaesthetic preferably is an amid based anaesthetic. Providing the anaesthetic as a first pharmaceutical liquid allows for a comparably comfortable administration of the therapeutic active ingredient which alone might be painful. In such configurations it might in many applications be particularly beneficial if the multi chamber syringe unit is embodied to sequentially provide the first and second pharmaceutical liquids. In particular, it might be beneficial if the multi chamber syringe unit is adapted to provide the anaesthetic in a first step and the therapeutic active ingredient in a second step.

In a second preferred embodiment, the first pharmaceutical liquid comprises a first therapeutic active ingredient and the second pharmaceutical liquid comprises a second therapeutic active ingredient. This can be useful for combinations of active ingredients which are used in one single therapy. In applications not covered by the claims it might also be useful to mix the two active ingredients and then injecting the mixture to a patient. Such administration might be particularly beneficial when the mixture of the two active ingredients is not stable for a certain time or when the two active ingredients do react with each other.

According to the invention, the first pharmaceutical liquid comprises an enzyme and the second pharmaceutical liquid comprises a therapeutic active ingredient. Thereby, the enzyme is a hyaluronidase. Often, the acceptance of the tissue where the therapeutic active ingredient is provided within a particular therapy is comparably low or insufficient especially for comparably large injection volumes. Thus, the tissue can only accept a certain amount of active ingredient whereas it would be advantageous for the success of the therapy to provide more active ingredient. In such situations it has been shown that in some cases the acceptance of the tissue can be increased when the tissue first receives an enzyme. For example, in such cases it can be beneficial to sequentially inject the enzyme and afterwards the therapeutic active ingredient. With the third embodiment of the invention this can be achieved in a safe, efficient and convenient manner.

The therapeutic active ingredients described herein can be small molecules. However, the therapeutic active ingredient preferably comprises a protein wherein the protein can be an antibody such as a monoclonal antibody. When using such a protein as therapeutic active ingredient the above mentioned effects can be particularly advantageous.

The first pharmaceutical liquid and the second pharmaceutical liquid together from a gel such as a hydrogel or complex upon mixing. A (hydro)gel or complex can be beneficial in therapy since it allows for continuously dispersing the therapeutic active ingredient over a comparably long time in a controlled release. Thus, by being a (hydro)gel or complex after mixing this embodiment of the multi chamber syringe unit allows for efficiently provide the active ingredient over a comparably long time at the place of injection.

In addition to the separation element, the multi chamber syringe unit may comprise a closing element arranged in the interior of the body closing the proximal chamber. The closing element can be a plunger such as an end plunger. Such a closing element allows for efficiently sealing the proximal chamber and preventing contamination.

Preferably, the multi chamber syringe unit comprises a needle connected to the distal opening of the distal end side of the body. Such a needle allows for efficiently injecting the first and second pharmaceutical liquids to the patient. Also, the multi chamber syringe unit can comprise an activation rod extending through the proximal end side of the body into the interior of the body. Such activation rod allows for conveniently activating and applying the multi chamber syringe unit.

In another embodiment not covered by the claims, a method of preparing a multi chamber syringe (preparation method) is provided. The multi chamber syringe has a longitudinal body with a side wall, a distal end side, a proximal end side opposite to the distal end side, an interior limited by the side wall between the distal end side and the proximal end side and a distal opening arranged in the distal end side for providing a liquid out of the multi chamber syringe. The preparation method comprises the steps of: sterilizing the multi chamber syringe; filling a first pharmaceutical liquid into the interior of the body adjacent to its distal end side; providing a middle plunger in the interior of the body of the multi chamber syringe such that a distal chamber and a proximal chamber are formed in the interior of the body; and filling a second pharmaceutical liquid into the proximal chamber of the interior of the body.

The various steps of the preparation method can be performed in the sequence as listed or in any other suitable sequence. For example, the step of providing a middle plunger in the interior of the body can be performed before filling any liquid. Thereby, the first pharmaceutical liquid can be filled from the distal end side and the second pharmaceutical liquid from the proximal end side after the middle plunger is arranged in the interior of the body.

Further, the various steps of the preparation method can all be performed at a single location or site. They can also be distributed to different locations which allows for a particularly efficient performance of single steps or combinations thereof. For example, the step of sterilizing the multi chamber syringe can be performed at the location where the syringe is filled. Or, it can be performed at the site of the manufacturer of the syringe such that the syringe is delivered in a pre-sterilized manner, e.g. as a so-called ready-to-fill syringe.

The preparation method according to the invention allows for efficiently providing a multi chamber syringe which can be used as a multi chamber syringe unit as described above. Like this, also the effects and benefits described above in connection with the multi chamber syringe unit according to the invention and the preferred embodiments thereof can efficiently be achieved.

Preferably, within the preparation method the first pharmaceutical liquid comprises any one of the group consisting of an anaesthetic, a first therapeutic active ingredient, a first hydrogel component and an enzyme such as hyaluronidase, and the second pharmaceutical liquid comprises a second hydrogel component and/or a second therapeutic active ingredient. The first pharmaceutical liquid can also comprise any combination of the components of the group.

Preferably, the preparation method further comprises the step of: providing a closing element such as an end plunger in the interior of the body of the multi chamber syringe such that the proximal chamber is closed.

Preferably, within the preparation method the multi chamber syringe is aligned with the distal end side of the body down during all steps of the method. Such an alignment allows for a particular efficient performing of the preparation method.

In another embodiment not covered by the claims, a therapeutic method is provided, comprising the steps of: obtaining a multi chamber syringe unit as described above and provided with a needle connected to the distal opening of the distal end side of the body; subcutaneously penetrating the needle of the multi chamber syringe unit; and activating the multi chamber syringe unit.

The term "activating" as used in this connection can relate to starting operation or operating the multi chamber syringe unit. It can particularly relate to providing the pharmaceutical liquids out of the body. In embodiments having a rod, such activation can be performed by pressing the rod into the interior of the body.

As described above with respect to plural aspects of the multi chamber syringe unit such therapy can be beneficial for plural reasons. In particular, by using the multi chamber syringe unit within the therapeutic method various effects and benefits described above in connection with the multi chamber syringe unit according to the invention and the preferred embodiments thereof can be achieved.

In one preferred embodiment of the therapeutic method, the first pharmaceutical liquid and the second pharmaceutical liquid of the multi chamber syringe unit are administered sequentially. In another preferred embodiment of the therapeutic method the first pharmaceutical liquid and the second pharmaceutical liquid of the multi chamber unit are administered concomitantly.

A first particular example of a therapeutic method comprises the steps of: obtaining a multi chamber syringe unit as described above provided with a needle connected to the distal opening of the distal end side of the body; subcutaneously penetrating the needle of the multi chamber syringe unit; and activating the multi chamber syringe unit.

A second particular example of a therapeutic method is the first particular example, wherein the first pharmaceutical liquid and the second pharmaceutical liquid of the multi chamber syringe unit are administered sequentially.

A third particular example of a therapeutic method is the first particular example, wherein the first pharmaceutical liquid and the second pharmaceutical liquid of the multi chamber syringe unit are administered concomitantly.

The aspects of the invention mentioned hereinbefore and other aspects will be apparent from and elucidated with reference to the embodiments described hereinafter.

### Brief Description of the Drawings

The multi chamber syringe unit according to the invention, the preparation method and the therapeutic method are described in more detail herein below by way of exemplary embodiments and with reference to the attached drawings, in which:
- Fig. 1: shows a side view on a first embodiment of a double chamber syringe as a first embodiment of a multi chamber syringe unit during concomitant liquid provision in a first embodiment of a therapeutic method;
- Fig. 2: shows a graph of the liquid provision of the therapeutic method of Fig. 1;
- Fig. 3: shows a side view on a second embodiment of a double chamber syringe as a second embodiment of a multi chamber syringe unit according to the invention during sequential liquid provision in a second embodiment of a therapeutic method not covered by the claims;
- Fig. 4: shows a graph of the liquid provision of the therapeutic method of Fig. 3;
- Fig. 5: shows a side view on a third embodiment of a double chamber syringe as a third embodiment of a multi chamber syringe unit during preparation in a first embodiment of a preparation method; and
- Fig. 6: shows a side view on a fourth embodiment of a double chamber syringe as a fourth embodiment of a multi chamber syringe unit during preparation in a second embodiment of a preparation method.

### Description of Embodiments

In the following description certain terms are used for reasons of convenience and are not intended to limit the invention. The terms "right", "left", "up", "down", "under" and "above" refer to directions in the figures. The terminology comprises the explicitly mentioned terms as well as their derivations and terms with a similar meaning. Also, spatially relative terms, such as "beneath", "below", "lower", "above", "upper", "proximal", "distal", and the like, may be used to describe one element's or feature's relationship to another element or feature as illustrated in the figures. These spatially relative terms are intended to encompass different positions and orientations of the devices in use or operation in addition to the position and orientation shown in the figures. For example, if a device in the figures is turned over, elements described as "below" or "beneath" other elements or features would then be "above" or "over" the other elements or features. Thus, the exemplary term "below" can encompass both positions and orientations of above and below. The devices may be otherwise oriented (rotated 90 degrees or at other orientations), and the spatially relative descriptors used herein interpreted accordingly. Likewise, descriptions of movement along and around various axes include various special device positions and orientations.

To avoid repetition in the figures and the descriptions of the various aspects and illustrative embodiments, it should be understood that many features are common to many aspects and embodiments. Omission of an aspect from a description or figure does not imply that the aspect is missing from embodiments that incorporate that aspect. Instead, the aspect may have been omitted for clarity and to avoid prolix description. In this context, the following applies to the rest of this description: If, in order to clarify the drawings, a figure contains reference signs which are not explained in the directly associated part of the description, then it is referred to previous or following description sections. Further, for reason of lucidity, if in a drawing not all features of a part are provided with reference signs it is referred to other drawings showing the same part. Like numbers in two or more figures represent the same or similar elements.

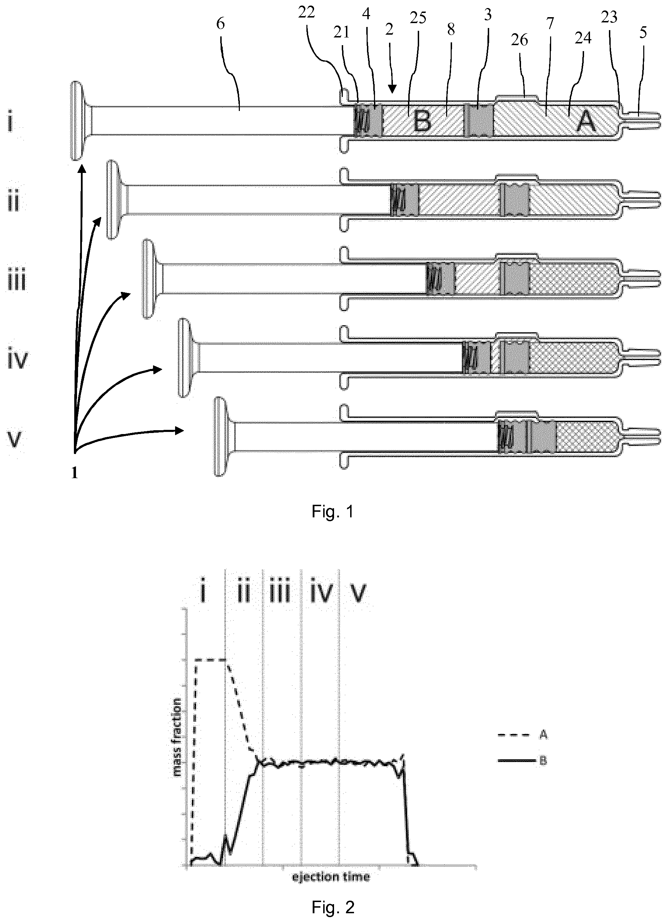

Fig. 1 shows a pre-filled double chamber syringe 1 as a first embodiment of a multi chamber syringe unit not covered by the claims. The syringe1 has a longitudinal hollow glass body 2 with a tubular side wall 21 surrounding an interior of the body 2. At its one end in a longitudinal direction, in Fig. 1 this is the right end, the body 2 has a distal end side 23 which is equipped with a distal opening passing over in a needle connector 5. At its other opposite end in the longitudinal direction, in Fig. 1 this is the left end, the body 2 has a proximal end side 22 having a main opening.

Between the distal end side 23 and the proximal end side 22 the side wall 21 is equipped with a longitudinal bulge 26 as a bypass arrangement. In the interior of the body 2 a middle plunger 3 as a separation element and an end plunger 4 as a closing element are arranged. Between the middle plunger 3 and the distal end side 23 a distal chamber 24 is formed in the interior of the body 2. Similarly, between the middle plunger 3 and the end plunger 4 a proximal chamber 25 is formed in the interior of the body 2. In the distal chamber 24 of the body 2 a first pharmaceutical liquid 7 (in Fig. 1 and Fig. 2 also referred to as A) and in the proximal chamber 25 of the body 2 a second pharmaceutical liquid 8 (in Fig. 1 and Fig. 2 also referred to as B) are arranged.

The syringe 1 is further equipped with an activating rod 6 which extends through the main opening at the proximal end 22 into the interior of the body 2. The activating rod 6 at its left end side has a finger rest and at its right end side is connected to the end plunger 4.

As visualized in Fig. 1 the syringe 1 is operated by pushing the activating rod 6 from left to right into the interior of the body 2. Thereby, in situation i the operation is initiated by applying a force to the finger rest of the rod 6 which, e.g. can be done by a thumb of a hand. As shown in situation ii the pressure inside the proximal chamber 25 is increased by the force acting on the rod 6 and the middle plunger 3 is moved from left to right into the direction of the distal end side 23 of the body 2 until it lies adjacent to or at the bulge 26. In this position a bypass channel is formed besides the middle plunger 3 by the bulge 26. By further advancing the activating rod 6 as shown in situation iii, the second pharmaceutical liquid 8 bypasses the middle plunger 3 and is transferred from the proximal chamber 25 into the distal chamber 24 wherein the middle plunger 3 is not moving. There, as shown in situation iv, the first pharmaceutical liquid 8 is mixed with the second pharmaceutical liquid 7. By still further advancing the activating rod 6, as shown in situation v, the middle plunger 3 moves further to the right hand side and pushes the mixture of first and second pharmaceutical liquids 7, 8 through the needle connector 5 out of the syringe 1.

In use in a therapeutic application a needle mounted to the needle connector 5 penetrates a target tissue, e.g. subcutaneously, and the syringe is activated, e.g. by the patient, as described above. Thereby, as can be seen in Fig. 2, even though in situation i some first pharmaceutical liquid is purely provided the first and second pharmaceutical liquids 7, 8 are to a major extent injected concomitantly.

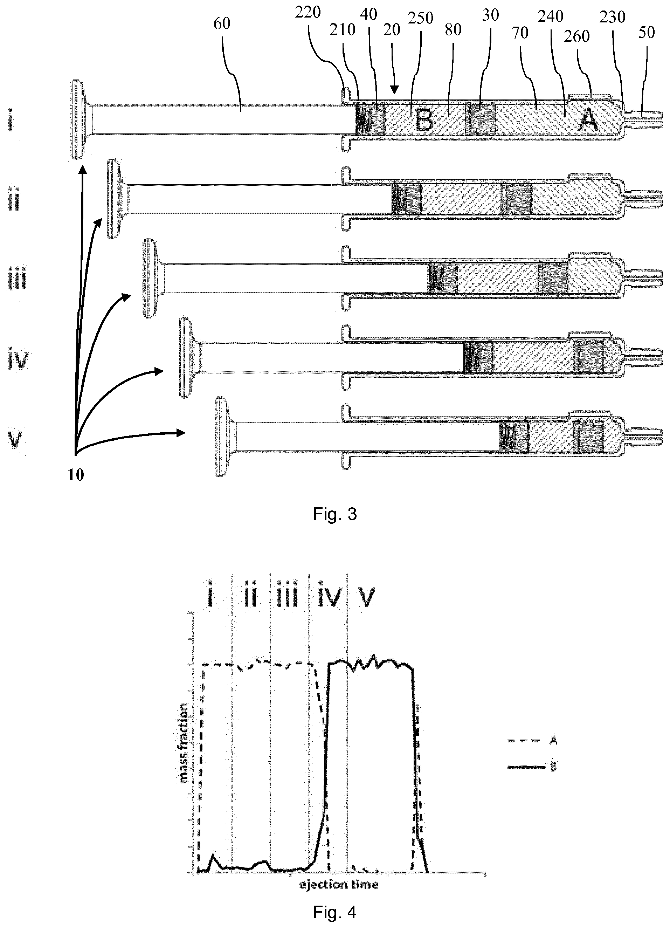

In Fig. 3 a pre-filled double chamber syringe 10 is shown as a second embodiment of a multi chamber syringe unit according to the invention. The syringe 10 has a longitudinal hollow glass body 20 with a tubular side wall 210 surrounding an interior of the body 20. At its one end in a longitudinal direction, in Fig. 3 this is the right end, the body 20 has a distal end side 230 which is equipped with a distal opening passing over in a needle connector 50. At its other opposite end in the longitudinal direction, in Fig. 3 this is the left end, the body 20 has a proximal end side 220 having a main opening.

Adjacent to the distal end side 230 the body 210 has a longitudinal bulge 260 as a bypass arrangement. In the interior of the body 20 a middle plunger 30 as a separation element and an end plunger 40 as a closing element are arranged. Between the middle plunger 30 and the distal end side 230 a distal chamber 240 is formed in the interior of the body 20. Similarly, between the middle plunger 30 and the end plunger 40 a proximal chamber 250 is formed in the interior of the body 20. In the distal chamber 240 of the body 20 a first pharmaceutical liquid 70 (in Fig. 3 and Fig. 4 also referred to as A) is arranged. In the proximal chamber 250 of the body 20 a second pharmaceutical liquid 80 (in Fig. 3 and Fig. 4 also referred to as B) is arranged.

The syringe 10 is further equipped with an activating rod 60 which extends through the main opening at the proximal end 220 into the interior of the body 20. The activating rod 60 at its left end side has a finger rest and at its right end side is connected to the end plunger 40.

As visualized in Fig. 3 the syringe 10 is operated by pushing the activating rod 60 from left to right into the interior of the body 20. Thereby, in situation i the operation is initiated by applying a force to the finger rest of the rod 60 which, e.g. can be done by a thumb of a hand. As shown in situation ii a pressure inside the proximal chamber 250 is increased and the middle plunger 30 is moved from left to right into the direction of the distal end side 230 of the body 20. During this movement of the middle plunger 30 the first pharmaceutical liquid 70 is provided through the needle connector 50 out of the distal chamber 240. As shown in situation iii the middle plunger 30 is further advanced to the right hand side and further first pharmaceutical substance 70 is dispensed. In situation iv the middle plunger 30 is moved as far to the right such that it lies adjacent to or at the bulge 260. In this position, a bypass channel is formed besides the middle plunger 30 by the bulge 260. The first pharmaceutical liquid 70 originally arranged in the distal chamber 240 of the body 20 is, to a large extent, already pushed out of the syringe 10 via the needle connector 50. The second pharmaceutical substance 80 starts to pass the middle plunger 30 via the bypass channel. By further advancing the activating rod 60, as shown in situation v the second pharmaceutical liquid 80 more and more bypasses the middle plunger 30 and is transferred from the proximal chamber 250 via the distal chamber 240 through the needle connector 50 out of the interior of the body 20.

In use, in a therapeutic application a needle mounted to the needle connector 50 penetrates a target tissue, e.g. subcutaneously, and the syringe 10 is activated, e.g. by the patient, as described above. Thereby, as shown in Fig. 4, the first and second pharmaceutical liquids 70, 80 are injected sequentially. In particular, since the bulge 260 is located close or adjacent to the distal end side 230 of the body 20 the distal chamber 240 is essentially emptied before the second pharmaceutical liquid 80 bypasses the middle plunger 30. Only in situation iv there is, for a comparably short time, a mixture of the first and second pharmaceutical liquids 70, 80 provided. However, it can efficiently be achieved that the first and second pharmaceutical liquids 70, 80 are administered one after the other.

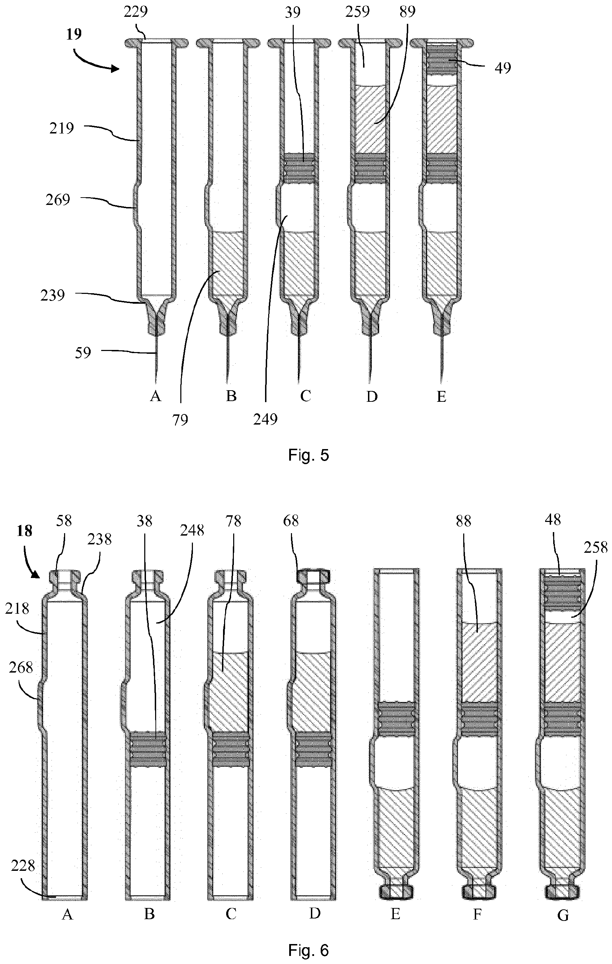

Fig. 5 shows a first embodiment of a method of preparing a staked-in needle pre-filled double chamber syringe 19 as a multi chamber syringe (preparation method). The syringe 19 has a longitudinal glass body 29 with a side wall 219 surrounding an interior. The body 29 has a lower distal end side 239, an upper proximal end side 229 opposite to the distal end side 239 and a distal opening arranged in the distal end side 239 for providing liquids out of the syringe 19. The distal opening is equipped with a needle 59. Between the distal end side 239 and the proximal end side 229 a longitudinal bulge 269 is formed in the side wall 219 of the body 29.

As shown in Fig. 5, the preparation method comprises five steps A through E. In step A the syringe 19 is positioned distal or front side down and prepared by sterilization. In step B a first pharmaceutical liquid 79 is filled into the interior of the body 219 via the main opening at the proximal end side 229. It is located adjacent to the distal end side 239 of the body 219. In step C a middle plunger 39 as separation element is forced into the interior of the body 219 and forwarded until it is located above the bulge 269. Thereby, a distal chamber 249 containing the first pharmaceutical liquid is formed in the interior of the body 219.

In step D a second pharmaceutical liquid 89 is filled in the interior of the body 219 via the main opening at the proximal end side 229. The second pharmaceutical liquid 89 lies on top of the middle plunger 39. In step E an end plunger 49 as closing element is placed into the interior of the body 219 via its main opening at the proximal end side 229. Thereby, a closed proximal chamber 259 containing the second pharmaceutical liquid 89 is formed in the interior of the body 219.

In Fig. 6 a second embodiment of a method of preparing a pre-filled double chamber syringe 18 as a multi chamber syringe (preparation method) is shown. The syringe 18 has a longitudinal glass body 28 with a side wall 218 surrounding an interior. The body 28 has a lower distal end side 238, an upper proximal end side 228 opposite to the distal end side 238 and a distal opening arranged in the distal end side 238 for providing liquids out of the body 28. The distal opening is equipped with a needle connector 58. Between the distal end side 238 and the proximal end side 228 a longitudinal bulge 268 is formed in the side wall 218 of the body 28.

As shown in Fig. 6, the preparation method comprises seven steps A through G. In step A the syringe 18 is positioned distal or front side up and prepared by sterilization. In step B a middle plunger 38 as separation element is forced bottom-up into the interior of the body 218 and forwarded until it is located below the bulge 268. Thereby, a distal chamber 248 is formed in the interior of the body 218 and above the middle plunger 38. In step C a first pharmaceutical liquid 78 is filled top-down into the interior of the body 218 via the distal opening of the distal end side 238. It is located inside the distal chamber 248 of the body 28. In step D a sealing cap 68 is mounted to the distal end side 238 of the body such that the distal opening is closed.

In step E the syringe 18 is turned around such that the distal side 238 of the body 28 is down and the proximal end side 228 is up. In step F a second pharmaceutical liquid 88 is filled in the interior of the body 218 via the main opening at the proximal end side 228 of the body 28. The second pharmaceutical liquid 88 lies on top of the middle plunger 38. In step G an end plunger 48 as closing element is placed into the interior of the body 218 via its main opening at the proximal end side 228. Thereby, a closed proximal chamber 258 containing the second pharmaceutical liquid 88 is formed in the interior of the body 218.

This description and the accompanying drawings that illustrate aspects and embodiments of the present invention should not be taken as limiting-the claims defining the protected invention. In other words, while the invention has been illustrated and described in detail in the drawings and foregoing description, such illustration and description are to be considered illustrative or exemplary and not restrictive. Various mechanical, compositional, structural, electrical, and operational changes may be made without departing from the scope of the claims. In some instances, well-known circuits, structures and techniques have not been shown in detail in order not to obscure the invention. Thus, it will be understood that changes and modifications may be made by those of ordinary skill within the scope of the following claims.

The disclosure also covers all further features shown in the Figs. individually although they may not have been described in the afore or following description. Also, single alternatives of the embodiments described in the figures and the description and single alternatives of features thereof can be disclaimed from the subject matter of the invention or from disclosed subject matter. The disclosure comprises subject matter consisting of the features defined in the claims or the exemplary embodiments as well as subject matter comprising said features.

Furthermore, in the claims the word "comprising" does not exclude other elements or steps, and the indefinite article "a" or "an" does not exclude a plurality. A single unit or step may fulfil the functions of several features recited in the claims. The mere fact that certain measures are recited in mutually different dependent claims does not indicate that a combination of these measures cannot be used to advantage. The terms "essentially", "about", "approximately" and the like in connection with an attribute or a value particularly also define exactly the attribute or exactly the value, respectively. The term "about" in the context of a given numerate value or range refers to a value or range that is, e.g., within 20%, within 10%, within 5%, or within 2% of the given value or range. Components described as coupled or connected may be electrically or mechanically directly coupled, or they may be indirectly coupled via one or more intermediate components. Any reference signs in the claims should not be construed as limiting the scope.

## Claims

1. Multi chamber syringe unit (1; 10; 18; 19) comprising
a longitudinal body (2; 20; 28; 29) with a side wall (21; 210; 218; 219), a distal end side (23; 230; 238; 239), a proximal end side (22; 220; 228; 229) opposite to the distal end side (23; 230; 238; 239), an interior limited by the side wall (21; 210; 218; 219) between the distal end side (23; 230; 238; 239) and the proximal end side (22; 220; 228; 229) and a distal opening arranged in the distal end side (23; 230; 238; 239) for providing a liquid out of the body (2; 20; 28; 29);
a separating element (3; 30; 38; 39) arranged in the interior of the body (2; 20; 28; 29) such that a distal chamber (24; 240; 248; 249) and a proximal chamber (25; 250; 258; 259) are formed in the interior of the body (2; 20; 28; 29), wherein the separating element (3; 30; 38; 39) seals the distal chamber (24; 240; 248; 249) from the proximal chamber (25; 250; 258; 259);
a bypass arrangement (26; 260; 268; 269) provided in the side wall (21; 210; 218; 219) of the distal chamber (24; 240; 248; 249) of the body (2; 20; 28; 29);
a first pharmaceutical liquid (7; 70; 78; 79) arranged in the distal chamber (24; 240; 248; 249) of the body (2; 20; 28; 29); and
a second pharmaceutical liquid (8; 80; 88; 89) arranged in the proximal chamber (25; 250; 258; 259) of the body (2; 20; 28; 29), wherein
the bypass arrangement (26; 260; 268; 269) is located adjacent to the distal end side (23; 230; 238; 239) of the body (2; 20; 28; 29), and
the body (2; 20; 28; 29) and the separating element (3; 30; 38; 39) are arranged such that the distal chamber (24; 240; 248; 249) is essentially emptied when the separating element (3; 30; 38; 39) is moved to the bypass arrangement (26; 260; 268; 269),
**characterized in that**
the first pharmaceutical liquid (7; 70; 78; 79) comprises an enzyme being hyaluronidase and the second pharmaceutical liquid (8; 80; 88; 89) comprises a therapeutic active ingredient, and
the first pharmaceutical liquid (7; 70; 78; 79) and the second pharmaceutical liquid (8; 80; 88; 89) together form a gel such as a hydrogel or complex upon mixing.

2. Multi chamber syringe unit (1; 10; 18; 19) according to claim 1, wherein the therapeutic active ingredient comprises a protein.

3. Multi chamber syringe unit (1; 10; 18; 19) according to claim 1 or 2, comprising a needle (58) connected to the distal opening of the distal end side (23; 230; 238; 239) of the body (2; 20; 28; 29).

## Patentansprüche

1. Mehrkammer-Spritzeneinheit (1; 10; 18; 19), umfassend
einen längsförmigen Körper (2; 20; 28; 29) mit einer Seitenwand (21; 210; 218; 219), einer distalen Endseite (23; 230; 238; 239), einer proximalen Endseite (22; 220; 228; 229) gegenüber der distalen Endseite (23; 230; 238; 239), einem Inneren, das durch die Seitenwand (21; 210; 218; 219) zwischen der distalen Endseite (23; 230; 238; 239) und der proximalen Endseite (22; 220; 228; 229) begrenzt ist, und einer distalen Öffnung, die in der distalen Endseite (23; 230; 238; 239) zum Bereitstellen einer Flüssigkeit aus dem Körper (2; 20; 28; 29) angeordnet ist;
ein Trennelement (3; 30; 38; 39), das im Inneren des Körpers (2; 20; 28; 29) angeordnet ist, so dass eine distale Kammer (24; 240; 248; 249) und eine proximale Kammer (25; 250; 258; 259) im Inneren des Körpers (2; 20; 28; 29) ausgebildet sind, wobei das Trennelement (3; 30; 38; 39) die distale Kammer (24; 240; 248; 249) von der proximalen Kammer (25; 250; 258; 259) abdichtet;
eine Umgehungsanordnung (26; 260; 268; 269), die in der Seitenwand (21; 210; 218; 219) der distalen Kammer (24; 240; 248; 249) des Körpers (2; 20; 28; 29) vorgesehen ist;
eine erste pharmazeutische Flüssigkeit (7; 70; 78; 79), die in der distalen Kammer (24; 240; 248; 249) des Körpers (2; 20; 28; 29) angeordnet ist; und
eine zweite pharmazeutische Flüssigkeit (8; 80; 88; 89), die in der proximalen Kammer (25; 250; 258; 259) des Körpers (2; 20; 28; 29) angeordnet ist, wobei
die Umgehungsanordnung (26; 260; 268; 269) sich angrenzend an der distalen Endseite (23; 230; 238; 239) des Körpers (2; 20; 28; 29) befindet, und
der Körper (2; 20; 28; 29) und das Trennelement (3; 30; 38; 39) angeordnet sind, so dass die distale Kammer (24; 240; 248; 249) im Wesentlichen entleert wird, wenn das Trennelement (3; 30; 38; 39) in die Umgehungsanordnung (26; 260; 268; 269) bewegt wird,
**dadurch gekennzeichnet, dass**
die erste pharmazeutische Flüssigkeit (7; 70; 78; 79) ein Enzym umfasst, das Hyaluronidase ist, und die zweite pharmazeutische Flüssigkeit (8; 80; 88; 89) einen therapeutischen Wirkstoff umfasst, und
die erste pharmazeutische Flüssigkeit (7; 70; 78; 79) und die zweite pharmazeutische Flüssigkeit (8; 80; 88; 89) bei einem Mischen zusammen ein Gel, wie etwa ein Hydrogel oder einen Komplex, bilden.

2. Mehrkammer-Spritzeneinheit (1; 10; 18; 19) nach Anspruch 1, wobei der therapeutische Wirkstoff ein Protein umfasst.

3. Mehrkammer-Spritzeneinheit (1; 10; 18; 19) nach Anspruch 1 oder 2, die eine Nadel (58) umfasst, die mit der distalen Öffnung der distalen Endseite (23; 230; 238; 239) des Körpers (2; 20; 28; 29) verbunden ist.

## Revendications

1. Unité de seringue à chambres multiples (1 ; 10 ; 18 ; 19) comprenant
un corps (2 ; 20 ; 28 ; 29) longitudinal doté d'une paroi latérale (21 ; 210 ; 218 ; 219), un côté d'extrémité distale (23 ; 230 ; 238 ; 239), un côté d'extrémité proximale (22 ; 220 ; 228 ; 229) opposé au côté d'extrémité distale (23 ; 230 ; 238 ; 239), un intérieur limité par la paroi latérale (21 ; 210 ; 218 ; 219) entre le côté d'extrémité distale (23 ; 230 ; 238 ; 239) et le côté d'extrémité proximale (22 ; 220 ; 228 ; 229) et une ouverture distale agencée dans le côté d'extrémité distale (23 ; 230 ; 238 ; 239) pour fournir un liquide hors du corps (2 ; 20 ; 28 ; 29) ;
un élément de séparation (3 ; 30 ; 38 ; 39) agencé à l'intérieur du corps (2 ; 20 ; 28 ; 29) de telle sorte qu'une chambre distale (24 ; 240 ; 248 ; 249) et une chambre proximale (25 ; 250 ; 258 ; 259) sont formées à l'intérieur du corps (2 ; 20 ; 28 ; 29), l'élément de séparation (3 ; 30 ; 38 ; 39) scellant la chambre distale (24 ; 240 ; 248 ; 249) par rapport à la chambre proximale (25 ; 250 ; 258 ; 259) ;
un agencement de dérivation (26 ; 260 ; 268 ; 269) fourni dans la paroi latérale (21 ; 210 ; 218 ; 219) de la chambre distale (24 ; 240 ; 248 ; 249) du corps (2 ; 20 ; 28 ; 29) ;
un premier liquide pharmaceutique (7 ; 70 ; 78 ; 79) agencé dans la chambre distale (24 ; 240 ; 248 ; 249) du corps (2 ; 20 ; 28 ; 29) ; et
un second liquide pharmaceutique (8 ; 80 ; 88 ; 89) agencé dans la chambre proximale (25 ; 250 ; 258 ; 259) du corps (2 ; 20 ; 28 ; 29),
l'agencement de dérivation (26 ; 260 ; 268 ; 269) étant situé à côté du côté d'extrémité distale (23 ; 230 ; 238 ; 239) du corps (2 ; 20 ; 28 ; 29) et
le corps (2 ; 20 ; 28 ; 29) et l'élément de séparation (3 ; 30 ; 38 ; 39) étant agencés de telle sorte que la chambre distale (24 ; 240 ; 248 ; 249) est essentiellement vidée lorsque l'élément de séparation (3 ; 30 ; 38 ; 39) est déplacé vers l'agencement de dérivation (26 ; 260 ; 268 ; 269),
**caractérisé en ce que**
le premier liquide pharmaceutique (7 ; 70 ; 78 ; 79) comprend une enzyme qui est la hyaluronidase et le second liquide pharmaceutique (8 ; 80 ; 88 ; 89) comprend un principe actif thérapeutique, et
le premier liquide pharmaceutique (7 ; 70 ; 78 ; 79) et le second liquide pharmaceutique (8 ; 80 ; 88 ; 89) forment ensemble un gel tel qu'un hydrogel ou un complexe lors du mélange.

2. Unité de seringue à chambres multiples (1 ; 10 ; 18 ; 19) selon la revendication 1, le principe actif thérapeutique comprenant une protéine.

3. Unité de seringue à chambres multiples (1 ; 10 ; 18 ; 19) selon la revendication 1 ou 2, comprenant une aiguille (58) reliée à l'ouverture distale du côté d'extrémité distale (23 ; 230 ; 238 ; 239) du corps (2 ; 20 ; 28 ; 29).
